# EUROPEAN PATENT APPLICATION

(11) **EP 2 322 169 A1**
(43) Date of publication of application: **18.05.2011**
(21) Application number: 09380176.9
(22) Date of filing: 13.11.2009
(51) Int. Cl.: A61K 31/202, A61K 45/06, A61P 11/00, A61P 43/00

(54) **Composition containing a DHA oil for use in the treatment of cystic fibrosis**

(71) Applicant: Laboratorios Casen-Fleet, S.L., 50180 Utebo ( Zaragoza) (ES)
(72) Inventor: Alvarez González, Juan Jesús, E 50180 Utebo (Zaragoza) (ES); Escobar Castro, Héctor, E 50180 Utebo (Zaragoza) (ES); Adalid Falcón, José M, E 50180 Utebo (Zaragoza) (ES); Ventura Barba, Antonio, E 50180 Utebo (Zaragoza) (ES); Alvarez Soto, Ana, E 50180 Utebo (Zaragoza) (ES); Navarro Lima, Angel, E 50180 Utebo (Zaragoza) (ES); Mulet Parada, Juan Manuel, E 50180 Utebo (Zaragoza) (ES); Rendón Ramírez, Omar, E 50180 Utebo (Zaragoza) (ES); Tabuenca Navarro, Daniel, E 50180 Utebo (Zaragoza) (ES)
(74) Representative: Ungria Lopez, Javier

(57) **Abstract**

Composition containing a DHA oil for use in the treatment of cystic fibrosis, the composition comprising, among other components:
◆ DHA (decosahexaenoic acid) (22:6n-3) in the form of, but not limited to, free fatty acid, monoglyceride, diglyceride, triglycerides or ethyl esthers at a percentage of up to 95% by weight;
◆ low content of EPA (eicosapentaenoic acid), estimated at an amount equal to or less than 2% by weight;
◆ low content of linoleic acid (18:2n-6) estimated at an amount equal to or less than 1 % by weight;
◆ low content of AA (arachidonic acid) estimated at an amount equal to or less than 1% by weight;

and may contain:
◆ vitamin C- and E-type antioxidants, and;
◆ amino acid methionine and nutrient choline,

having marine algae or fish as an origin,
being useful for the treatment of cystic fibrosis in newborn and youth.

## Description

### OBJECT OF THE INVENTION

The following invention, as set forth in the heading of the present specification, relates to a composition containing DHA (decosahexaenoic acid) (22:6n-3) oil for use in the treatment of cystic fibrosis, having as objectives to attack exocrine pancreas inflammation and impairment, delaying or improving them, as well as attack cystic fibrosis in newborn and youth, reducing its effects, which allows to improve the quality of life of ill persons and, surprisingly, being able to even eliminate them.

### BACKGROUND OF THE INVENTION

Cystic Fibrosis (CF) is a genetic disease, of autosomal recessive inheritance, present in the whole world, but more frequent in the Caucasian race, where one in 25 people is a healthy heterozygous carrier.

The incidence in our environment is estimated at about 1 in 4.500 live newborn (NB). The disease is caused by single gene mutations located at the long arm of chromosome 7, which codes for a protein, called transmembrane conductance regulator protein (CFTR: Cystic Fibrosis Transmembrane Regulator).

This protein acts as chlorine channel, controlled by cAMP (cyclic Adenosine Monophosphate), which regulates directly chloride particles movements and indirectly sodium and water movements, giving rise to the production of an anomalous and thick mucus in all organs, specially in the lungs, pancreas, intestine, liver, sweat glands and deferent ducts in male, interfering with its functionalism.

Generally CF has been considered as a genetic disorder which gives rise to a severe disease early in life, the most common manifestations of which are intestinal malabsorption with chronic diarrhea, malnutrition and pneumopathy, but it is currently known as a complex disorder producing a wide range of clinical expressions than can emerge at any age and also in an atypical way.

The digestive involvement is practically total and the three primary functions are disrupted: digestion, absorption and motility, being the main symptom malabsorption.

Among the more prevalent manifestations are meconium ileus, occurring in 10-20% of NB as the pattern of presentation for the disease, motility disorders suggesting dysmotility or the presence of obstructive phenomena such as the distal intestinal obstruction syndrome (DIOS) the prevalence of which increases parallel with age and resulting from the impaction of the viscose intestinal contents and, finally, rectal prolapse which occurs in 20% of the cases and is usually due to steatorrhea.

Pancreatic Failure (PF) occurs in 85-90% of patients and develops prematurely, generally during the first year, determining maldigestion-malabsorption with steatorrhea and nutrient loss, defective absorption of fat soluble vitamins and trace elements, being the inadequate ponderal gain together with the existence of abdominal strain and pale, fetid and oily abundant depositions the fundamental clinical manifestation. Usually indirect methods are utilized for the study of the pancreatic function such as the determination of fat in feces or the concentration of pancreatic enzymes in feces (elastase).

Also, relatively frequent forms of clinical presentation are the severe ions loss through sweat with electrolytic depletion and disturbances of the acid-base balance of acute (hyponatremic dehydration) or chronic (hypochloremic alkalosis with prostration, anorexia and impairment set of symptoms) onset and the syndrome of anemia-hypoproteinemia-edema occurring especially in the first months. Other manifestations are colonopathy as a consequence of injuries to the surface of the intestinal mucosa resulting in progressive fibrosis and stenosis and that has been related to the use of pancreatic supplements at high dosages.

The hepatobiliary involvement occurring in 18-37% of the patients with a varied spectrum of disturbances (microvesicle, lithiasis, cholestasis, steatosis, choledocal stenosis, etc.), although the typical injury is focal biliary cirrhosis which can progress into a multilobular cirrhosis over the course of the disease. Diabetes, whose incidence and prevalence increase with age and is caused by a progressive insulinopenia secondary to gradual loss of pancreatic tissue and acute or recurrent pancreatitis that generally appears in patients without PF or retaining a residual function.

The respiratory manifestations may appear at early ages with symptoms of bronchospasm, distress or recurrent pneumonia, leading to the onset of diffuse bronchiectasis. Infections by *Haemophilus influenzae, Staphylococcus aureus* and *Pseudomonas aeruginosa* are prevalent and by very harmful germs such as *Burkholderia cepacia, Stenotrophomona maltophilia* and *Achromobacter xylosoxidans* in advanced stages of the disease.

The treatment of the disease is based on 4 fundamental milestones: -1 - treatment of infection, inflammation and mucociliary clearance. -2- Replacement therapy with pancreatic enzymes and nutrition control. -3- Respiratory physiotherapy to eliminate thick secretions. And -4- Control and treatment of the associated diseases hepatopathy and diabetes.

On the other hand, different documents may be considered wherein are described compositions for the treatment of cystic fibrosis or conditions related thereto, and, thus, we may quote the document ES 2059986 T3 wherein a composition comprising a microbial lipase and a mammalian pancreatic extract as active component is described, which is used in the treatment of chronic or acute pancreatitis, duodenal neoplasms, poor digestion after pancreas or stomach resection or cystic fibrosis.

In the document ES 2136315 T3 is described a set or kit comprising a perfluorocarbon liquid and a microparticulate medicament dispersed in a breathable gas in order to form a gaseous suspension for use in the treatment of a pulmonary state or condition in a mammal, and namely the pulmonary state being a cystic disease.

In the document ES 2170769 T3 the use of an antibiotic in the manufacture of a medicament to facilitate clearance of pulmonary mucus and attack cystic fibrosis is described.

Likewise, the following patent documents may be considered:
◆ US 6,180,671 and US 6,552,081 wherein a "method for treating disorders in which DHA levels are affected" is described, which is based on its administration, in patients with the DHA levels affected by a problem at the CFTR gene, and is particularly useful against, for example, cystic fibrosis.
◆ US 2004/0127567 wherein likewise, a "method for treating disorders in which DHA levels are affected" is described, useful particularly against cystic fibrosis.

### DESCRIPTION OF THE INVENTION

In the present specification is described a composition containing a DHA (decosahexaenoic acid) oil for use in the treatment of cystic fibrosis, the composition comprising, among other components:
◆ DHA (decosahexaenoic acid) (22:6n-3) in the form of, but not limited to, free fatty acid, monoglyceride, diglyceride, triglycerides or ethyl esthers at a percentage of up to 95% by weight;
◆ low content of EPA (eicosapentaenoic acid) (20:5n-3), estimated at an amount equal to or less than 2% by weight;
◆ low content of LA (linoleic acid) (18:2n-6) estimated at an amount equal to or less than 1 % by weight;
◆ low content of AA (arachidonic acid) estimated at an amount equal to or less than 1% by weight;
and may contain:
◆ vitamin C- and E-type antioxidants; and
◆ amino acid methionine and nutrient choline,
having marine algae or fish as an origin, such that said composition is useful for the treatment of cystic fibrosis, particularly, in newborn and youth.

In this way, an attempt to reduce the effects caused by cystic fibrosis is made, improving the quality of life of ill persons and being able to even eliminate its effects.

This compound should be administrated orally. The utilized dosage would be less than 6 g/day.

### Example 1

Based on the described composition, a 5,000 g compound was prepared with the object of treating a group of 25 rats afflicted with cystic fibrosis, subjecting them to a 3 g/day treatment.

Within ten days it could be appreciated how the disease was stabilized and within 40 days a substantial improvement was verified by having reduced the effects thereof.

Following, the dose was reduced to 1 g/day and within 60 days, surprisingly, could be verified that in a group of 6 rats the effects had totally disappeared and in the remainder the effects had been substantially reduced, that is, the disease had not only been stabilized but even, surprisingly, had been eliminated.

### Example 2

Based on the described composition, a 5,000 g compound was prepared with the object of treating a group of 20 rats afflicted with cystic fibrosis, by subjecting a group of 10 rats to a 2 g/day treatment and to the other group of 10 rats to a 3 g/day treatment.

Within ten days it could be appreciated how in the group of 10 rats treated with 2 g/day the disease had been stabilized and in the group of 10 rats treated with 3 g/day the improvement had been slightly greater and within 40 days a substantial improvement was verified in both groups by having reduced the effects thereof.

Following, the dose was reduced to 1 g/day and within 60 days, surprisingly, could be verified that in two rats from the group of 10 rats treated, previously, with 2 g/day and 3 rats from the group of 10 rats treated, previously, with 3 g/day, the effects had totally disappeared and in the remainder the effects had been substantially reduced, that is, the disease had not only been stabilized but, even, surprisingly, had been eliminated.

## Claims

1. COMPOSITION CONTAINING A DHA OIL FOR USE IN THE TREATMENT OF CYSTIC FIBROSIS, the composition comprising, among other components:
◆ DHA (decosahexaenoic acid) (22:6n-3) in the form of, but not limited to, free fatty acid, monoglyceride, diglyceride, triglycerides or ethyl esthers at a percentage of up to 95% by weight;
◆ low content of EPA (eicosapentaenoic acid), estimated at an amount equal to or less than 2% by weight;
◆ low content of linoleic acid (18:2n-6) estimated at an amount equal to or less than 1 % by weight;
◆ low content of AA (arachidonic acid) estimated at an amount equal to
or less than 1 % by weight;
and may contain:
◆ vitamin C- and E-type antioxidants, and;
◆ amino acid methionine and nutrient choline,
having marine algae or fish as an origin,
**characterized in that** said composition with a content of DHA oil is useful for the treatment of cystic fibrosis in newborn and youth.

2. USE, according to claim 1, **characterized in that** the dose of the composition is administrated to newborn and youth orally.

3. USE, according to claim 1, **characterized in that** the dose administrated to newborn and youth is of up to 6 g/day.
